(19) Europäisches Patentamt
European Patent Office
Office européen des brevets

(11) Veröffentlichungsnummer: **0 152 027**
**B1**

(12) # EUROPÄISCHE PATENTSCHRIFT

(45) Veröffentlichungstag der Patentschrift: **11.04.90**

(51) Int. Cl.$^5$: **C 07 D 307/58**

(21) Anmeldenummer: **85101035.5**

(22) Anmeldetag: **01.02.85**

(54) **Verfahren zur Herstellung von 3-Methyl-2-buten-4-olid(4-methyl-2-(5H)-furan-2-on).**

(30) Priorität: **03.02.84 DE 3403793**

(43) Veröffentlichungstag der Anmeldung:
**21.08.85 Patentblatt 85/34**

(45) Bekanntmachung des Hinweises auf die Patenterteilung:
**11.04.90 Patentblatt 90/15**

(84) Benannte Vertragsstaaten:
**AT BE CH DE FR GB IT LI LU NL**

(56) Entgegenhaltungen:
**EP-A-0 086 457**
**EP-A-0 124 725**
**DE-B-2 154 439**
**DE-C-1 276 029**
**GB-A-1 148 043**

**JOURNAL OF THE CHEMICAL SOCIETY, CHEMICAL COMMUNICATIONS, 1979, Seite 982, London, GB; Y. INOUE et al.: "Reaction of Methylenecyclopropanes with carbon dioxide catalysed by palladium (O) complexes. Synthesis of five-membered lactones"**

(73) Patentinhaber: **Studiengesellschaft Kohle mbH**
**Kaiser-Wilhelm-Platz 1**
**D-4330 Mülheim/Ruhr (DE)**

(72) Erfinder: **Binger, Paul, Dr.**
**Lembkestrasse 4**
**D-4330 Mülheim/Ruhr (DE)**
Erfinder: **Weintz, Hans-Joachim**
**Lembkestrasse 5**
**D-4330 Mülheim/Ruhr (DE)**

(74) Vertreter: **von Kreisler, Alek, Dipl.-Chem. et al**
**Deichmannhaus am Hauptbahnhof**
**D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 152 027 B1**

**Beschreibung**

Gegenstand der Erfindung ist ein Verfahren zur Herstellung von 3-Methyl-2-buten-4-olid (4-Methyl-2(5H)-furan-2-on) gemäß obigen Patentansprüchen.

Katalysierte Cycloadditionen von Methylencyclopropanen mit $CO_2$ an Palladium(O)-Verbindungen zu Dihydrofuran-2-onen bzw. Methylen-tetrahydrofuran-2-onen sind bisher nur mit an der Doppelbindung mono- oder disubstituierten Methylencyclopropanen beschrieben worden. Am Dreirung substituierte Methylencyclopropane gehen diese Codimerisierung nicht ein. (Lit.: Y. Inoue, T. Hibi, M. Satake, H. Hashimito, J. Chem. Soc. Chem. Comm. *1979,* 982). Entsprechende Reaktionen mit dem Grundkörper Methylencyclopropan (1) sind in der Literatur unbekannt. Nach einer Privatmitteilung von Y. Inoue sollen sie sehr uneinheitlich verlaufen.

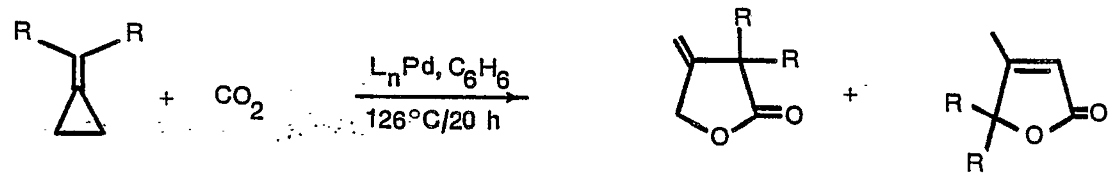


(1)

$R=CH_3$ ; $-(CH_2)_5-$

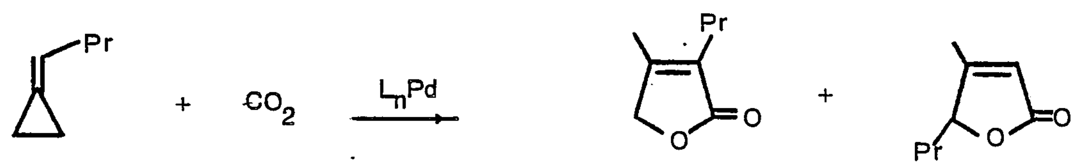


(2)

$Pr=C_3H_7$

| | |
|---|---|
| DMSO | Dimethylsulfoxid |
| DMF | Dimetylformamid |
| $Pd(DBA)_2$ | Bis(dibenzylidenaceton)palladium |
| DBA | Benzylidenaceton |

Es wurde nun gefunden, daß man auch die katalytische Codimerisierung von Methylencyclopropan (1) mit $CO_2$ an Pd(O)-Verbindungen glatt durchführen kann. Man erhält z.B. bei 150—200°C mit $CO_2$— Drücken von 30—80 bar das 3-Methyl-2-butan-4-olid (2) in bis zu 85% Ausbeute, wobei pro g-Atom Pd bis zu 2000 Mol 1 umgesetzt werden. Neben der Codimerisierung erfolgt auch noch Cyclodimerisierung von 1 zu 3 und Alkylierung von 2 zu 4—8, wobei 1 unter dem Einfluß des Katalysators als Alkylierungsmittel fungiert. Andere Produkte treten praktisch nicht auf.

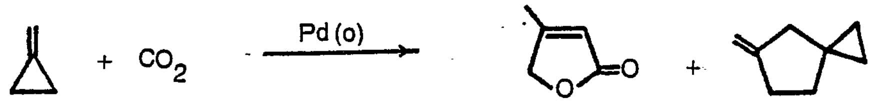

(GL. 3)

1 2 3

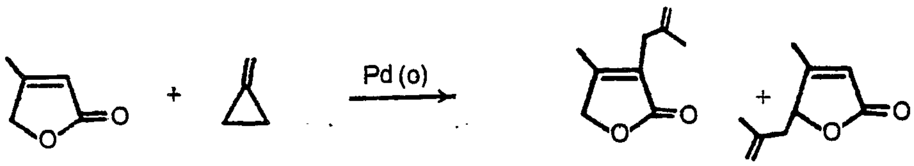

(GL.4)

2 1 4 5

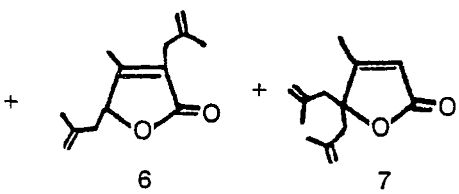

6 7

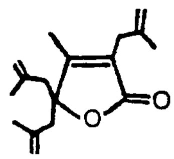

8

Als Katalysatoren eignen sich alle Palladium(O)-Verbindungen, die im Reaktionsgemisch bis 200°C stabil sind. Dazu gehören:

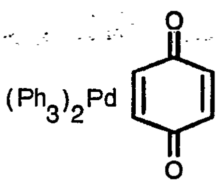 ; 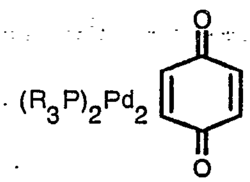 ; 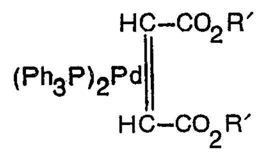

R=nBu. Ph. $C_6H_{11}$ Phenoxy: 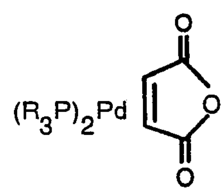 ;

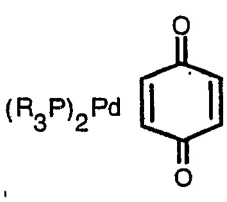 ; 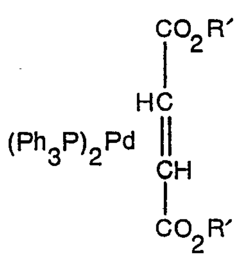 ; 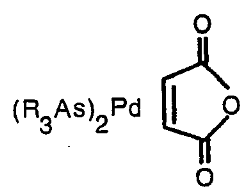 ;

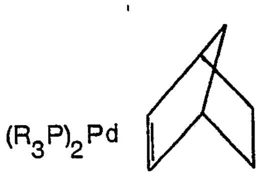 R= Me, $C_6H_{11}$; $(R_3P)_2Pd\cdot AcN$;

R = Alkyl, Phenyl, Alkoxy, Phenoxy;

$(R_2P)_2$ Pd..Äthylen, R = Me, $C_6H_{11}$;

$(DBA)_2Pd$; $Pd_2(DBA)_3\cdot S$;

$(R_2P)_2PdCOD$, R = Me, $C_6H_{11}$;

$(Ph_3P)_2Pd_2(DBA)_3$; $[R_3P]_3Pd_2(DBA)_3$;

$Pd(PPh_3)_4$; $Pd[PMe_2Ph]_4$;

$Pd[Me_3]_4$; $Pd[PMePh_2]_4$; $Pd[PEt_3]_4$;

$Pd[PBu_3]_4$; $Pd[P(iPr_3)]_{2und3}$;

$Pd[P(C_6H_{11})_3]_{2und3}$; $Pd[P(tBu)_2Ph]_2$;

$Pd[P(CH_2Ph)_3]_3$; $Pd[PPh(tBu)_2]_2$;

$Pd[P(tBu)_3]_2$;

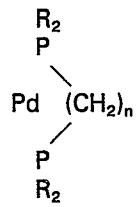

n = 1 3; $Pd[P(OR)_3]_4$

Besonders reaktiv sind 1:2 bis 1:8-Gemische einer Pd(O)-Verbindung, wie z.B. Bis(dibenzylidenaceton)-palladium[$Pd(DBA)_2$], (Lit.: T. Ukai, H. Kawazuma, Y. Ishidi, J. J. Bennet und J. A. Ibers, J. Organomet. Chem. *65*, 253 (1974)), Bis(cyclooctadien-1,5)-palladium oder Tris(norbornen)-palladium (Lit.: M. Green, J. A. K. Howard, J. L. Spencer und P. G. A. Stone, J. Chem. Soc. Chem. Comm. *1975*, 449) mit einem Trialkylphosphan, Triarylphosphan, Trialkylphosphit, Triarylphosphit oder einem zweizähnigen Bis(organylphosphoryl)-1,2-ethan oder -1,3-propan.

Auch in situ hergestellte Pd(O)-Katalysatoren sind hochaktiv. Diese stellt man durch Reduktion einer Pd(II)-verbindung, wie z.B. Palladiumacetylacetonat, Palladium(II)-nitrat, Palladium(II)-chlorid, Palladium(II)-acetat mit einem Metallorganyl, wie z.B. Lithiumbutyl, Ethylmagnesiumchlorid oder Diethylethoxialuminium in Gegenwart der oben erwähnten Phosphane bzw. Diphosphane her. Auch hochaktives Magnesium bzw. die Kombination Magnesium/Anthracen (Lit.: H. Bönnemann, B. Bogdanovic, DE—PS 32 05 550.1 (Priorität 17.2.82); Eur. Pat. 83 101 246.3 (10.2.83), Studiengesellschaft Kohle mbH.) sind als Reduktionsmittel gut geeignet. Eine besonders erfolgreiche Quelle für in situ hergestellte Katalysatoren ist die Kombination von $\eta^3$Allyl-$\eta^5$cyclopentadienyl-palladium (Lit.: Y. Tatsumo, T. Yoshida und S. Otsura, Inorg. Synth. *19*, 220 (1979)) zusammen mit den oben genannten Phosphanen bzw. Diphosphanen.

Die Herstellung der oben genannten Pd(O)-Verbindungen bzw.-Katalysatoren ist in P. Maitlis "The Organic Chemistry of Palladium" Band 1 (New York 1971) oder in P. M. Maitlis und M. J. H. Russell, Comprehensive Organomet. Chem. Vol. 6, S. 243—265 Pergamon Press 1982 beschrieben.

Erfindungsgemäß wird das Verfahren bei Temperaturen von 100—200°C, und Drücken von 10—110 bar, bevorzugt bei 30—80 bar durchgeführt. Dabei ist die Verwendung eines Lösungsmittels, wie z.B. Aromaten (Benzol, Toluol, Xylol), Ether (Diethylether, Tetrahydrofuran oder Dioxan) sowie anderer polarer, aprotischer Lösungsmittel (Acetonitril, Dimethylsulfoxid, Dimethylformamid) nützlich. Es kann aber auch ohne Lösungmittel gearbeitet werden. Besonders hohe Ausbeuten an 2 unter weitgehender Verhinderung der Bildung von 3 und 4—8 erhält man, wenn man unter Vorlage von $CO_2$ und eines Teils des Lösungsmittels (DMF) das Katalysator/1-Gemisch mit einer Geschwindigkeit von 1—20 ml/min. prop 200 ml Reaktionsvolumen bei 150—200°C einspritzt. Das erfindungsgemäße Verfahren kann diskontinuierlich, alternativ aber auch kontinuierlich durchgeführt werden.

Das 3-Methyl-2-buten-4-olid 2 ist als Ausgangsverbindung in der Naturstoffsynthese interessant. So kann man ausgehend von 2 z.B. die Seitenkette von α-Tocopherol aufbauen (Lit.: M. Schmid, R. Barner, Helv. Chim. Acta, *62*, 464 (1979).

Auch Rosefuran, der Geruchsträger des bulgarischen Rosenöls ist aus 2 zugänglich (Lit.: D. R. Gedge u. G. Pattenden, Tetrahedron Letters *1977*, 4443).

Arbeitsvorschriften

Beispiel 1

Ein 200 ml V4A-Stahlautoklav wird mit 15 ml DMF beschickt, auf 165°C (Innentemperatur) aufgeheizt und 40 bar $CO_2$ aufgepreßt. Aus einem unter 25 bar $N_2$-Druck stehenden Vorratsgefäß wird eine Lösung von 0,5 g (0,28 mmol) ($\eta^3$-Allyl)-($\eta^5$-cyclopentadienyl)palladium, 0,27 g (1,1 mmol) Triphenylphosphan und 20,6 g (0,381 mol) 1 in 90 ml DMF mittels einer Dosierpumpe in ca. 1 h zugepumpt. Die Zupumpgeschwindigkeit wird so gewählt, daß die Wärmetönung 5°C nicht übersteigt; der $CO_2$-Druck wird auf 40 bar konstant gehalten. Anschließend wird eine weitere Stunde gerührt. Man füllt 145,7 g schwarze Reaktionslösung aus, von der alle flüchtigen Bestandteile (142,7 g) bis Sdp. 60°/0,001 mbar abdestilliert werden; 1,9 g zäher, schwarzer Rückstand. Anschließende fraktionierende Destillation über eine 60 cm Vigreux-Kolonne ergibt nach 109 g DMF vom Sdp. ca. 50°C/7 mbar 31 g 2 (97% GC; 80% d.Th.) vom Sdp. 131—132°C/7 mbar Rest (GC): 2,1% 4, 0,9% 5. Aus dem Rückstand werden durch einfache Destillation 6 g farblose Flüssigkeit von Sdp. 41—60°C/0,001 mbar gewonnen. Zusammensetzung (GC): 37,1% 4; 46,4% 5; 4,7% 6; 6,7% 7; 1,2% 9; Rest (3.9%) 4 Peaks.

Beispiel 2

Analog Beispiel 1 wird der Autoklav mit 20 ml DMF beschickt, auf 150°C aufgeheizt und 50 bar $CO_2$ aufgepreßt. Dazu wird eine Lösung von 0,49 g (0,85 mmol) $Pd(DBA)_2$, 0,89 g (3,4 mmol) Triphenylphosphan und 16 g (297 mmol) 1 in 80 ml DMF in 10 Minuten gepumpt, wobei die Temperatur auf max. 190°C und der

Druck kurzfristig auf 65 bar steigt. Nach 30 minütiger Nachreaktion ist die Reaktion beendet. Man erhält bei der fraktionierenden Destillation 25,8 g (85%) 2.

Beispiel 3

Analog Beispiel 1 wird der Autoklav mit 20 ml DMSO beschickt, auf 180°C aufgeheizt und 30 bar $CO_2$ aufgepreßt. Dazu wird in 2 h eine Lösing von 0,45 g (ca. 0,5 mmol) Tetrakis(triphenylphosphan)palladium, 0,52 g (ca. 2 mmol) Triphenylphosphan mit 16 g (297 mmol) 1 in 180 ml DMSO zugepumpt, wobei der $CO_2$-Druck konstant bei 30 bar gehalten wird. Hierbei erhält man 18,9 g (65%) 2.

Beispiel 4

Analog Beispiel 1 erhält man aus 1 g (0,47 mmol) $\eta^3$-Allyl-$\eta^5$-cyclopentadienylpalladium, 0,97 g (3,76 mmol) Triphenylphosphan, 9,3 g (172 mmol) 1 in 20 ml DMF bei 16 bar $CO_2$ (Reaktionstemperatur 152°C) 12,1 g Produkt mit der GC-Analyse: 48% 2; 21% 4; 10% 5, 11% 6; 5% 7 und 4% 8.

Beispiel 5

Analog 1 erhält man aus 0,49 g (0,85 mmol) $Pd(DBA)_2$, 0,54 g (3,4 mmol) Triisopropylphosphan und 18,3 g (339 mmol) 1 in 30 ml DMF und bei 16 bar $CO_2$ (Reaktionstemperatur: 154°C) 14,3 g Produkt mit der GC-Analyse: 10,2% 2; 15% 3; 7% 4; 2% 5; 40% 6; 2% 7 und 7% 8; Rest (27%) Vielzahl unbekannter Verbindungen zwischen 0,5 und 2%.

Beispiel 6

In einen 200 ml V4A-Stahlautoklaven werden nacheinander eingefüllt: 0,38 g (0,66 mmol) $Pd(DBA)_2$, 0,72 g (2,64 mmol) Triphenylphosphan, 20 ml Toluol und bei −78°C 12,8 g (237 mmol) 1. Man preßt bei Raumtemperatur 40 bar $CO_2$ auf und erhitzt den Autoklaven unter Schütteln 20 h bei 130°C (Maximaldruck: 85 bar). Die dunkelbraune Reaktionslösung wird ausgefüllt und fraktionierend destilliert. Man erhält nach 17,6 g farbloser Flüssigkeit vom Sdp. bis 30°C (12 mbar) mit der GC-Analyse: 97,7% Toluol und 2,3% 3 (ca. 0,4 g (3,2%)); 10,9 g schwach gelb gefärbte Flüssigkeit vom Sdp. 35—70°C/0,001 mbar; GC-Analyse: 23,6% 2, 14,5% 4; 8,2% 5; 12,4% 6; 2,5% 7 und 6,8% 8; Rest 31,6%: Vielzahl unbekannter Verbindungen zwischen 0,5 und 2%. 1,2 g zäher, schwarzer Rückstand.

Beispiel 7

Analog Beispiel 2 erhält man aus 0,13 g (0,61 mmol) $\eta^3$-Allyl-$\eta^5$-cyclopentadienylpalladium, 0,33 g (1,22 mmol) Triphenylphosphan und 10 g (0,85 mmol) 1 in 20 ml DMF bei 32 bar $CO_2$ nach 15 h bei 144°C 8 g Produkt mit der GC-Analyse: 32% 2; 22% 4; 8% 5; 6% 6; 11% 7 und 5% 8; Rest (16%): Vielzahl unbekannter Verbindungen zwischen 0,5 und 2,4%.

Beispiel 8

Analog Beispiel 2 erhält man aus 0,14 g (0,66 mmol) $\eta^3$-Allyl-$\eta^5$-cyclopentadienylpalladium, 0,36 g (1,32 mmol) Triphenylphosphan und 18 g (335 mmol) 1 in 20 ml DMSO bei 32 bar $CO_2$ nach 18 h bei 140°C. 10,5 g Produkt vom Sdp. 55—70°C/0,001 mbar mit der GC-Analyse: 3% 4; 9% 6; 4% 7 und 69% 8; Rest (15%): Vielzahl unbekannter Verbindungen zwischen 0,5 und 2%.

**Patentansprüche**

1. Verfahren zur Herstellung von 3-Methyl-2-buten-4-olid durch Codimerisation von Methylencyclopropan mit Kohlendioxid ($CO_2$) in Gegenwart von organischen Komplexverbindungen von Übergangsmetallen, dadurch gekennzeichnet, daß man die Codimerisierung bei 100—200°C in Gegenwart von Palladium(O)-Verbindungen, die im Reaktionsgemisch bis 200°C stabil sind, und mit $CO_2$ bei Drücken von 10—110 bar in Gegenwart eines Lösungsmittels durchführt.

2. Verfahren nach Anspruch 1, dadurch gekennzeichnet, daß man die Palladium(O)-verbindungen in situ herstellt.

3. Verfahren nach Anspruch 1 und 2, dadurch gekennzeichnet, daß man bei Temperaturen von 150—200°C arbeitet.

4. Verfahren nach Anspruch 1—3, dadurch gekennzeichnet, daß man bei Drücken von 30—80 bar arbeitet.

5. Verfahren nach Anspruch 1—4, dadurch gekennzeichnet, daß man in einem polaren, aprotischen Lösungsmittel, wie z.B. Dimethylsulfoxid (DMSO) oder Dimethylformamid (DMF) arbeitet.

6. Verfahren nach Anspruch 1—5, dadurch gekennzeichnet, daß man eine Lösung des Katalysators (Konzentration 1—10 mmol/l Lösungsmittel) mit Methylencyclopropan (Konzentration 2—5 mol/l Lösungsmittel) bei Reaktionstemperatur in das Lösungsmittel/$CO_2$-Gemisch (200 ml Reaktionsvolumen) mit einer Geschwindigkeit vom 1—20 ml/min. ohne zusätzliche Kühlung einspritzt.

**Revendications**

1. Procédé pour préparer le 3-méthyl-2-butène-4-olide par codimérisation du méthylènecyclopropane

avec l'anhydride carbonique ($CO_2$) en présence de composés complexes organiques de métaux de transition, procédé caractérisé en ce qu'on conduit la codimérisation à 100—200°C en présence de composés du palladium(O), qui sont stables dans le mélange réactionnel jusqu'à 200°C, et avec $CO_2$ sous des pressions de 10 à 110 bars, en présence d'un solvant.

2. Procédé selon la revendication 1, caractérisé en ce qu'on prépare sur place ("in situ") les composés du palladium(O).

3. Procédé selon les revendications 1 et 2, caractérisé en ce qu'on travaille à des températures de 150 à 200°C.

4. Procédé selon les revendications 1 à 3, caractérisé en ce qu'on travaille sous des pressions de 30 à 80 bars.

5. Procédé selon les revendications 1 à 4, caractérisé en ce qu'on travaille dans un solvant aprotique polaire comme, par exemple, le diméthylsulfoxyde (DMSO) ou le diméthylformamide (DMF).

6. Procédé selon les revendications 1 à 5, caractérisé en qu'on injecte sans refroidissement supplémentaire une solution du catalyseur (concentration de 1 à 10 mmoles/litre de solvant) avec du méthylènecyclopropane (concentration 2 à 5 moles/litre de solvant) à la température de réaction dans le mélange solvant/$CO_2$ (200 ml de volume réactionnel) à une vitesse ou un débit de 1 à 20 ml/min.

**Claims**

1. A process for the production of 3-methyl-2-buten-4-olide by codimerization of methylene cyclopropane with carbon dioxide ($CO_2$) in the presence of organic complex compounds of transition metals, characterized in that the codimerization is carried out at 100 to 200°C in the presence of palladium(O) compounds, which are stable in the reaction mixture up to 200°C, and with $CO_2$ under pressures of 10 to 110 bar in the presence of a solvent.

2. A process as claimed in claim 1, characterized in that the palladium(O) compounds are prepared *in situ*.

3. A process as claimed in claims 1 and 2, characterized in that it is carried out at temperatures of 150 to 200°C.

4. A process as claimed in claims 1 to 3, characterized in that it is carried out under pressures of 30 to 80 bar.

5. A process as claimed in claims 1 to 4, characterized in that it is carried out in a polar aprotic solvent, such as for example dimethyl sulfoxide (DMSO) or dimethyl formamide (DMF).

6. A process as claimed in claims 1 to 5, characterized in that a solution of the catalyst (concentration 1—10 mmol/l solvent) is sprayed with methylene cyclopropane (concentration 2—5 mol/1 solvent) into the solvent/$CO_2$ mixture (200 ml reaction volume) at the reaction temperature without additional cooling at a rate of 1—20 ml/min.